# EUROPEAN PATENT SPECIFICATION

(11) **EP 1 930 044 B1**
(45) Date of publication and mention of the grant of the patent: **23.08.2017**
(21) Application number: 07014671.7
(22) Date of filing: 26.07.2007
(51) Int. Cl.: A61M 31/00

(54) **Two-phase vaginal lavage**
Zweiphasige Vaginalspülung
Lavage vaginal à deux phases

(43) Date of publication of application: 11.06.2008
(73) Proprietor: Livi, Elisabetta, 50019 Sesto Fiorentino (FI) (IT)
(72) Inventor: Livi, Elisabetta, 50019 Sesto Fiorentino (FI) (IT)
(74) Representative: Asensio, Raffaella Consuelo

(56) References cited:
- EP-A- 0 956 858
- WO-A-2007/029047
- US-A- 3 211 149
- US-A1- 2007 110 805

## Description

It is known that the vaginal lavages are used in gynecology to clean, to disinfect and to medicate the inside parts of the female genital organ. Currently the vaginal lavages are principally with watery base. The watery base vaginal lavages have the work to deterge and to oppose the action of the bacteria by the action of the medicinal active principles present into the watery base. Said lavages, based onto watery solvents, have the problem of the scant permanence onto the vaginal tissue, such as the watery base provides to deterge the vaginal secretions and to slide out of the parts where it is distributed. Consequently, also the medicinal active principles have a short time of permanence onto the parts infected. So have fundamental importance for a good effect of the vaginal lavage to make a lavage with watery base followed to that with oil or gel base, or to the medicament indicated to the gynaecologist , such as the oil or the gel-oil solution favours the permanence of the active medical principle onto the vaginal tissue.

Two-phase vaginal lavage are known, as for instance the subject-matter described in document WO 2007/029047. This document refers to a gynaecological and safety device for a treatment composed of one or two pistons and a capsule which includes the active material. The pistons are used to dispense the solutions of active material. However, the cited device has high production costs.

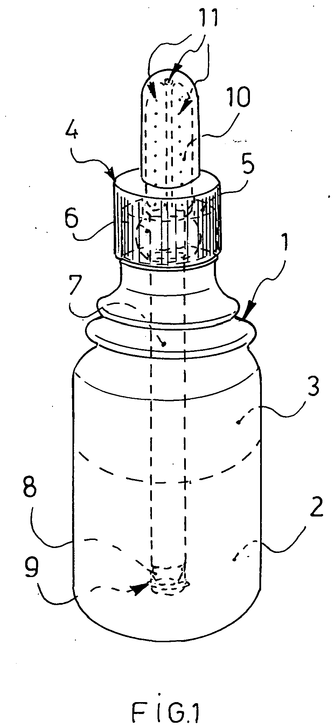

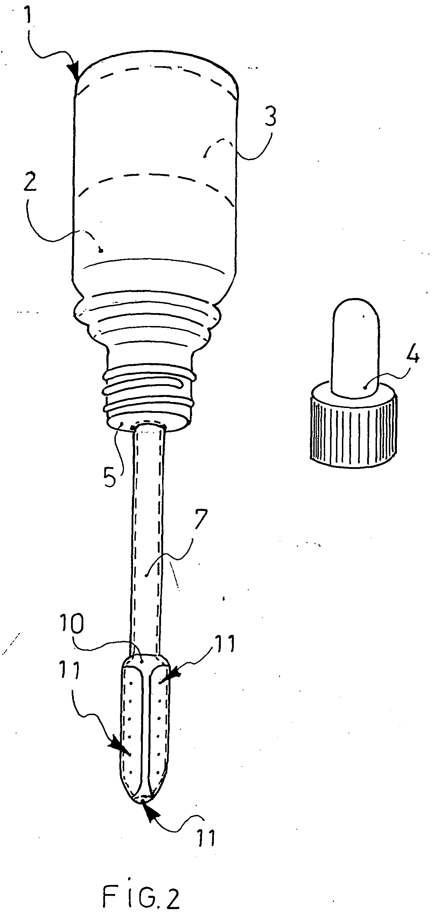

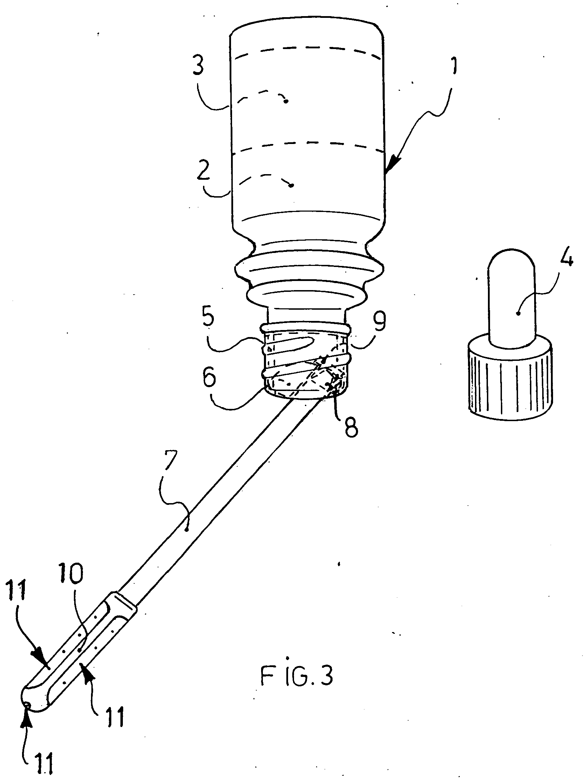

Subject of the invention is a new type of two-phase vaginal lavage so to have in a sole packaging both a watery vaginal lavage than a vaginal lavage with oil or gel-oil base. The invention so drawing up determines a big reduction of the industrial costs such as it permits to have in a sole packaging both the types of lavages, i.e. whether the vaginal lavage with watery base that one with oil or gel-oil base. The invented subject essentially consists of a container with water solution in the low part and with oil or gel-oil solution in the upper part, said upper solution that floats over the under water solution, without mixing with it. In upsetting of the packaging the water solution remains in the lower layer and the oil solution or with oil-gel to the upper layer. A container with exit of the fluxes in the upper part, with the same upside-down, distributes so first the watery solution and after, per gravitational action and/or for pressure onto the container, distributes the oil or oil-gel solution. In this way with a sole application the distribution into the vagina of the watery solution with active medical principles deterges and disinfects all the vaginal secretions and, immediately after, the oil or oil-gel solution is distributed so to have, before of other eventual secretions and onto the clean and disinfected vaginal tissue, the application of the oil film with the medical active principles distributed onto the vaginal tissue in optimum way. In this way the therapeutic effect is prolonged such as the contact time of the medicinal substances distribute with the invented method is of over twenty-four hours in comparison with the duration of about ten minutes obtained to the simply watery lavages. The invention is illustrated in a merely indicative and not limiting way in the drawings of sheets 1, 2 and 3. In particular in sheet 1 the figure 1 is perspective view of the invention in closed phase. In sheet 2 the figure 2 is view of the invention with the cannula extracted. In sheet 3 figure 3 is perspective view of the invention prompt to be used. In a detailed description and in an actuation embodiment not exclusive, the invention consists of a bottle 1 in plastic material. Inside said bottle is present a watery solution 2 with the medical active principles in the quantity indicated on the base of the use of the vaginal lavage. Upper the watery solution is present the oil or oil-gel solution 3 with the medicinal active principles. Said oil or oil gel solution 3 with the medicinal active principles can be realized on the base of each solution having medicinal active principles provided that it can float without mixing into the under watery solution with medical principles 2. When the bottle 1 is in upsetting, i.e. during the use phase, the watery solution stays in low position with upper the layer of the solution with oil or oil-gel base. The bottle 1 has un plug with seal 4 where in its inside is present a subplug 5 having at its inside a hemisphere 6. In a central position, and free to translate if pull toward or push to the inside of the bottle 1 and passing for the subplug 5 and the hemipsher 6, is present a cannula 7. Said cannula 7 is equipped with a valve 8 and with an abutment 9 in the final part, whereas in the initial part provides an anatomic distributor 10 with holes 11. The cannula 7, the hemisphere 6 and the subplug 5 form a device to realize a rotate horizontally pivot slanting cannula. In working the invented apparatus provides, to the user of the product, the unscrewing of the plug 4 to the bottle and the extraction of the cannula 7 from the inside part of the bottle body, with subsequent positioning of the rotate horizontally pivot slanting cannula into the part of the vaginal tissue to bedew. The fluid placed inside the bottle 1 comes to exit to the same body bottle 1 to flow through the cannula 7 and from this to exit by the holes 11. For the particular and characteristic disposition of the watery base vaginal lavage 2 and of the oil or oil-gel base lavage 3 in the upsetting bottle, it is determined the ordinate exit from the same bottle before of the watery base liquid and subsequent of the oil base liquid. The exit of the liquids comes both for gravitational action than for the pressure onto the flexible part of the bottle 1. So it is realized in a sole container a vaginal lavage that provides, in succession, the action of the lavage with aqueous base followed in the last phase to the distribution of the oil or oil-gel base lavage. In the realization, the details of working, the dimensions, the shape and the used substances used in the invention can, however, change without going out to the present invention, on condition that they maintain the essential characteristic to have one or more oil or oil-gel layers that float without mixing with one or more layers with prevalently watery base with a cannula that distributes the fluids contained in the bottle 1 on the sequence given to the chemical-physical characteristic of the used substances.

## Claims

1. Two-phase vaginal lavage comprising a watery solution (2) with medical active principles and an oil or gel-oil solution (3) with medical active principles; wherein the oil or gel-oil solution (3) floats over the watery solution without mixing with the under watery solution (2), and wherein both the solutions (2, 3) are contained in a sole bottle (1) with a flexible part and having an outlet equipped with a cannula (7), which comes into contact with watery phase (2) when the bottle is in the upset position to determinate first the outlet from the bottle of the watery solution (2) and subsequently the outlet of the oil or gel-oil solution (3), both with medical active principles, **characterized in that** the outlet from the bottle of the watery solution (2) and subsequently the outlet of the oil or gel-oil solution (3) is determined by gravitational action.

2. Two-phase vaginal lavage, as to the previous claim, **characterized in that** inside a sole bottle (1) with a flexible part is contained a solution (3) consisting of one or more oil or gel-oil layers that float without mixing over another solution (2), consisting of one or more layers prevalently watery, with a cannula (7) that distributes the fluids contained in the bottle (1).

3. Two-phase vaginal lavage, as to the previous claims, as a sole packaging wherein
i. said watery solution (2) cleans from vaginal secretions and disinfects the vaginal tissue, and
ii. said oil or oil-gel solution (3) contains the medical active principles for prolonging the therapeutic effect.

## Patentansprüche

1. Zweiphasige Vaginalspülung, die eine wässrige Lösung (2) mit medizinischen Wirkstoffen und eine Öl- oder Gelöllösung (3) mit medizinischen Wirkstoffen umfasst; wobei die Öl- oder Gelöllösung (3) über die wässrige Lösung ohne Vermischung mit der darunterliegenden Lösung (2) schwimmt und wobei beide Lösungen (2, 3) in einer einzigen Flasche (1) mit einem flexiblen Teil und mit einem mit einer Kanüle (7) ausgestatteten Auslass enthalten sind, wobei die Kanüle mit der wässrigen Phase (2) in Berührung kommt, wenn sich die Flasche in der umgekehrten Stellung befindet, um zunächst den Abfluss aus der Flasche der wässrigen Lösung (2) und anschließend den Abfluss der Öl- oder Gelöllösung (3), beide mit medizinischen Wirkstoffen, zu bestimmen, **dadurch gekennzeichnet, dass** der Abfluss aus der Flasche der wässrigen Lösung (2) und anschließend der Abfluss der Öl- oder Gelöllösung (3) durch Gravitationswirkung bestimmt wird.

2. Zweiphasige Vaginalspülung nach dem vorhergehenden Anspruch, **dadurch gekennzeichnet, dass** innerhalb einer einzigen Flasche (1) mit einem flexiblen Teil eine Lösung (3) enthalten ist, die aus einer oder mehreren Öl- oder Gelölschichten besteht, die ohne Vermischung über eine andere Lösung (2) schwimmen, die aus einer oder mehreren überwiegend wässrigen Schichten besteht, mit einer Kanüle (7), die die in der Flasche (1) enthaltenen Flüssigkeiten verteilt.

3. Zweiphasige Vaginalspülung nach den vorstehenden Ansprüchen als einzige Verpackung, wobei
i. die wässrige Lösung (2) aus vaginalen Sekreten das Vaginalgewebe reinigt und desinfiziert, und
ii. die obengenannte Öl- oder Öl-Gel-Lösung (3) die medizinischen Wirkstoffe zur Verlängerung der therapeutischen Wirkung enthält.

## Revendications

1. Lavage vaginal à deux phases comprenant une solution aqueuse (2) avec des principes actifs médicaux et une solution d'huile ou de gel-huile (3) avec des principes actifs médicaux; où la solution d'huile ou d'huile de gel (3) flotte sur la solution aqueuse sans se mélanger avec la sous-jacente solution aqueuse (2), et où les deux solutions (2, 3) sont contenues dans une seule bouteille (1) avec une partie flexible et avec une sortie équipée d'une canule (7) qui entre en contact avec la phase aqueuse (2) lorsque la bouteille est dans une position à l'envers pour déterminer d'abord la sortie de la bouteille de la solution aqueuse (2) et ensuite la sortie de la solution d'huile ou d'huile de gel (3), toutes les deux avec des principes actifs médicaux, **caractérisé en ce que** la sortie de la bouteille de la solution aqueuse (2) et ensuite la sortie de la solution d'huile ou de gel-huile (3) est déterminée par une action gravitationnelle.

2. Lavage vaginal à deux phases selon la revendication précédente, **caractérisé en ce qu'**à l'intérieur d'une seule bouteille (1) avec une partie flexible se trouve une solution (3) constituée d'une ou plusieurs couches d'huile ou de gel-huile qui flottent sans se mélanger sur une autre solution (2), consistant en une ou plusieurs couches prévalentement aqueuses, avec une canule (7) qui distribue les fluides contenus dans la bouteille (1).

3. Lavage vaginal à deux phases selon les revendications précédentes, comme un seul emballage où
i. ladite solution aqueuse (2) nettoie des sécrétions vaginales et désinfecte le tissu vaginal, et
ii. ladite solution d'huile ou de gel d'huile (3) contient les principes actifs médicaux pour prolonger l'effet thérapeutique.
